# EUROPÄISCHE PATENTSCHRIFT

(11) **EP 0 639 955 B1**
(45) Veröffentlichungstag und Bekanntmachung des Hinweises auf die Patenterteilung: **15.05.1996**
(21) Anmeldenummer: 94908948.6
(22) Anmeldetag: 01.03.1994
(51) Int. Cl.: A61B 17/58, F16B 23/00

(54) **SCHRAUBE ZUM FESTLEGEN EINES MARKRAUMNAGELS**
SCREW FOR SECURING AN INTRAMEDULLARY NAIL
VIS PERMETTANT DE FIXER UN CLOU INTRAMEDULLAIRE

(30) Priorität: 11.03.1993 DE 4307633
(43) Veröffentlichungstag der Anmeldung: 01.03.1995
(73) Patentinhaber: Pennig, Dietmar, Dr. med., D-50935 Köln (DE)
(72) Erfinder: Pennig, Dietmar, Dr. med., D-50935 Köln (DE)
(74) Vertreter: Habbel, Hans-Georg, Dipl.-Ing.
(86) Internationale Anmeldenummer: DE9400228
(87) Internationale Veröffentlichungsnummer: WO9420039

(56) Entgegenhaltungen:
- DE-A- 2 246 274
- US-A- 4 723 541
- US-A- 5 116 337

## Beschreibung

Die Erfindung bezieht sich auf eine Schraube zum Festlegen eines Markraumnagels gemäß dem Oberbegriff des Hauptanspruches.

Das Festlegen von Markraumnägeln mittels Schrauben ist z. B. aus der DE-A-22 46 274, die zur Bildung des Oberbegriffs des Anspruchs 1 herangezogen werden ist, bekannt. Hierbei werden Schrauben eingesetzt, die auf einem Teil ihrer Schaftlänge nahe dem Schraubenkopf mit einem Außengewinde ausgerüstet sind. Das Gewinde ist selbstschneidend und schraubt sich in die Cortikalis.

Nur auf einem Teil ihrer Länge mit Außengewinde ausgerüstete Schrauben sind aus der US 44 63 753 bekannt, wobei die beiden Gewindebereiche dieser Schraube dazu dienen, eine Kompressionsschraube zu schaffen, die die beiden durch eine Fraktur getrennten Knochenteile aufeinanderzu zieht. Der Gewindesinn der beiden voneinander entfernten Gewindebereiche ist dabei gleich gerichtet, und diese Schraube wird nicht in Verbindung mit einem Markraumnagel eingesetzt.

Demgegenüber liegt der Erfindung die Aufgabe zugrunde, eine Schraube zur Festlegung eines Markraumnagels zu schaffen, die in einfachster Weise den Markraumnagel festlegen soll und leicht entfernbar sein soll.

Diese der Erfindung zugrundeliegende Aufgabe wird durch die Lehre des Hauptanspruches gelöst.

Vorteilhafte Ausgestaltungen sind in den Unteransprüchen erläutert.

Mit anderen Worten ausgedrückt, wird eine Schraube vorgeschlagen, diean der Außenseite des Schraubenkopfes ein Außengewinde aufweist, dessen Gewindesinn aber entgegengesetzt dem Gewindesinn ist, den das Gewinde auf dem Schraubenschaft aufweist. Hierdurch ist es möglich, das zum Herausziehen der Schraube auf die Außenseite des Schraubenkopfes ein mit Innengewinde ausgerüsteter Schraubenschlüssel angesetzt wird, durch dessen Drehung, die entgegengesetzt der Drehung gerichtet ist, die beim Einschrauben der Schraube ausgeübt wird, nunmehr die Schraube aus dem Knochen herausgeschraubt werden kann.

Diese Drehrichtung könnte auch durch den Inbusschraubendreher erzielt werden, aber bei einem solchen Inbusschraubendreher kann kein Zug auf die Schraube ausgeübt werden, so daß das Herauslösen der eingewachsenen Schraube aus dem Knochen so gut wie unmöglich ist.

Das Außengewinde des Schraubenkopfes beginnt nicht an der Oberseite des Schraubenkopfes, sondern etwas zurückversetzt, so daß ein zylindrischer Vorsprung im Bereich des Schraubenkopfes entsteht, der das Ansetzen des Schraubendrehers mit Innengewinde erleichtert.

Zwischen der Unterseite des Schraubenkopfes und dem Außengewinde des Schraubenschaftes ist ein Freiraum vorhanden, der sich nach der Größe der Schraube und dem Einsatzzweck der Schraube richtet.

Ein Ausführungsbeispiel der Erfindung wird nachfolgend anhand der Zeichnungen erläutert.

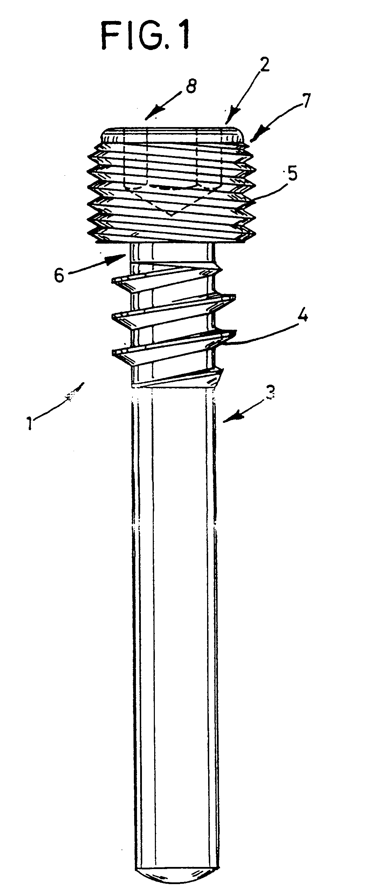

Die Zeichnungen zeigen in
- Fig. 1: eine neue Schraube und in

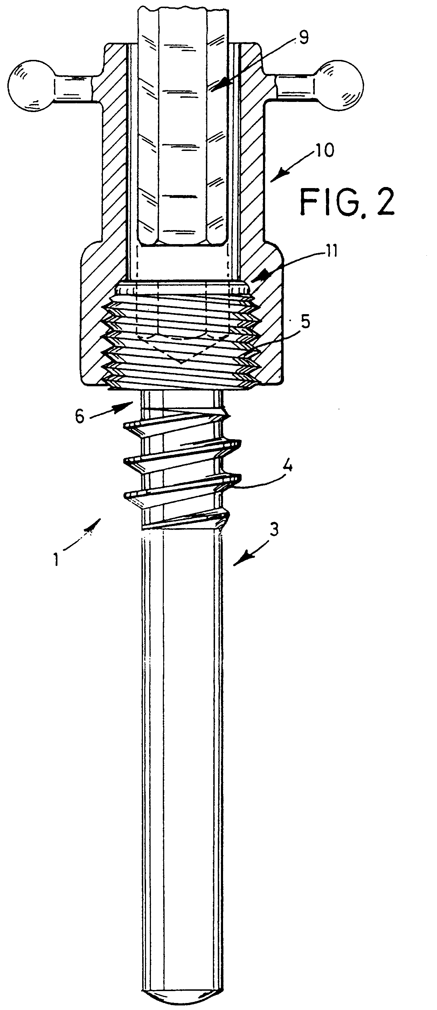

- Fig. 2: eine Schraube mit zwei Schraubendrehern.

In den Zeichnungen ist eine Schraube 1 dargestellt, die einen Schraubenkopf 2 und einen Schraubenschaft 3 aufweist. Der größte Bereich des Schraubenschaftes ist gewindeles ausgebildet, und nur in Nähe der Unterseite des Schraubenkopfes 2 ist ein Schraubenschaftgewinde 4 vorgesehen. Beim Einsatz der Schraube 1 zum Festlegen des Marknagels schraubt sich dieses Schraubenschaftgewinde 4 in die Kortikalis des Knochens ein.

Der Schraubenkopf 2 ist als Inbuskopf 8 ausgebildet, kann aber auch mit einem Kreuzschlitz ausgerüstet sein, so daß durch Ansetzen eines entsprechenden Schraubendrehers 9 ein Einschrauben der Schraube möglich ist.

An der Außenseite des Schraubenkopfes ist ein Schraubenkopfgewinde 5 vorgesehen, dessen Gewindesinn entgegengesetzt dem Gewindesinn des Schraubenschaftgewindes 4 ausgerichtet ist, so daß bei Aufdrehen eines mit einem entsprechenden Innengewinde ausgerüsteten Schraubendrehers 10 und nach Anlage einer Schulter 11 am Schraubenkopf 2 nunmehr ein Rückdrehen der Schraube 1 aus dem Knochen des Patienten möglich ist. Hierbei kann gleichzeitig ein entsprechender Zug auf die Schraube 1 aufgebracht werden, so daß ein gutes Lösen der Schraube 1 aus dem Knochen möglich wird.

Wie die Zeichnung verdeutlicht, wird zwischen der Oberkante des Schraubenkopfgewindes 5 und der Oberseite des Schraubenkopfes 2 ein zylindrischer gewindefreier Bereich 7 vorgesehen, der ein leichtes Einsetzen des Schraubendrehers 10 auf das Schraubenkopfgewinde 5 ermöglicht, auch dann wenn sich der Schraubenkopf innerhalb des Patienten - also nicht sichtbar - befindet.

Zwischen der Unterseite des Schraubenkopfes 2 und dem Schraubenkopfgewinde 4 ist ein gewindefreier Bereich 6 vorhanden, der sich nach der Größe der Schraube 1 und dem Einsatzzweck richtet.

## Patentansprüche

1. Schraube (1) zum Festlegen eines Markraumnagels, mit einem Schraubenkopf (2) und einem mit Außengewinde (4) versehenen Schraubenschaft (3), wobei das Schraubenschaftgewinde (4) nahe am Schraubenkopf (2) nur auf einem Teil der Länge des Schraubenschaftes (3) vorgesehen ist und der Schraubenkopf (2) als Inbus- oder Kreuzschlitzschraubenkopf ausgebildet ist, dadurch gekennzeichnet, daß der Schraubenkopf (2) auf seiner Außenseite ein Außengewinde(5) aufweist, dessen Gewindesinn entgegengesetzt dem Gewindesinn des Schraubenschaftgewindes (4) ist.

2. Schraube nach Anspruch 1, dadurch gekennzeichnet, daß das Schraubenkopfgewinde (5) im Abstand vom freien Ende des Schraubenkopfes (2) beginnt und der Außendurchmesser des Schraubenkopfes (2) hier etwa dem Kerndurchmesser des Schraubenkopfgewindes (5) entspricht.

3. Schraube nach Anspruch 1 oder 2, dadurch gekennzeichnet, daß zwischen dem Schraubenschaftgewinde (4) und der Unterseite des Schraubenkopfes (2) ein gewindefreier Bereich (6) vorgesehen ist.

4. Schraube nach einem der vorhergehenden Ansprüche, dadurch gekennzeichnet, daß sich die Gewindetiefe des Schraubenschaftgewindes (4) zum gewindelosen Schraubenschaft (3) hin verringert.

## Claims

1. A screw (1) for securing a medullary space pin, comprising a screw head (2) and a screw shank (3) provided with an external thread (4), the screw shank thread (4) in the vicinity of the screw head (2) being provided over only part of the length of the screw shank (3) and the screw head (2) being constructed as a hexagon socket screw head or a cross-recess screw head, characterized in that the screw head (2) comprises an external thread (5) on its outside, the thread direction of which runs counter to the thread direction of the screw shank thread (4).

2. A screw according to claim 1, characterized in that the screw head thread (5) begins at a distance from the free end of the screw head (2) and the outer diameter of the screw head (2) here corresponds to approximately the core diameter of the screw head thread (5).

3. A screw according to claim 1 or claim 2, characterized in that between the screw shank thread (4) and the underside of the screw head (2) there is provided a thread-free area (6).

4. A screw according to any one of the preceding claims, characterized in that the thread depth of the screw shank thread (4) diminishes towards the thread-less screw shank (3).

## Revendications

1. Vis (1) pour la fixation d'un clou intramédullaire, avec une tête de vis (2) et un corps de vis (3) pourvu d'un filet extérieur (4), dans laquelle le filet de corps de vis (4) proche de la tête de vis (2) n'est prévu que sur une partie de la longueur du corps de vis (3) et la tête de vis (2) est conçue comme une tête de vis inbus ou à fente cruciforme, caractérisée en ce que la tête de vis (2) présente sur son côté extérieur un filet extérieur (5) dont le sens de filetage est opposé au sens du filet de corps de vis (4).

2. Vis selon la revendication 1, caractérisée en ce que le filet de tête de vis (5) débute à distance de l'extrémité libre de la tête de vis (2) et le diamètre extérieur de la tête de vis (2) correspond approximativement au diamètre de l'âme du filet de tête de vis (5) à cet endroit.

3. Vis selon la revendication 1 ou 2, caractérisée en ce qu'il est prévu entre le filet de corps de vis (4) et le côté inférieur de la tête de vis (2) une zone sans filet (6).

4. Vis selon l'une ou l'ensemble des revendications précédentes, caractérisée en ce que la profondeur de filetage du filet de corps de vis (4) diminue en direction du corps de vis (3) non fileté.
